(19) Europäisches Patentamt
European Patent Office
Office européen des brevets

(11) Veröffentlichungsnummer: **0 187 216**
**B1**

(12) # EUROPÄISCHE PATENTSCHRIFT

(45) Veröffentlichungstag der Patentschrift: **27.04.88**

(51) Int. Cl.⁴: **C 07 C 49/227**

(21) Anmeldenummer: **85113942.8**

(22) Anmeldetag: **02.11.85**

(54) **Verfahren zur Herstellung von halogenierten 3,3-Dimethyl-5-hexen-2-onen.**

(30) Priorität: **13.11.84 DE 3441370**

(43) Veröffentlichungstag der Anmeldung:
**16.07.86 Patentblatt 86/29**

(45) Bekanntmachung des Hinweises auf die Patenterteilung:
**27.04.88 Patentblatt 88/17**

(84) Benannte Vertragsstaaten:
**AT BE CH DE FR GB IT LI NL**

(56) Entgegenhaltungen:
**EP-A-0 095 047**
**FR-A-2 005 165**

(73) Patentinhaber: **BAYER AG, Konzernverwaltung RP Patentabteilung, D-5090 Leverkusen 1 Bayerwerk (DE)**

(72) Erfinder: **Lantzsch, Reinhard, Dr., Am Buschhäuschen 51, D-5600 Wuppertal (DE)**

**EP 0 187 216 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft ein neues Verfahren zur Herstellung von halogenierten 3,3-Dimethyl-5-hexen-2-onen.

Es ist bereits bekannt, daß halogenierte 3,3-Dimethyl-5-hexen-2-one z. B. hergestellt werden können, indem man Vinylaldehyde mit 2-Methylbutan-3-on in Gegenwart von Halogenwasserstoff säuren umsetzt. Diese Synthesen sind teuer, da die Vinylaldehyde schwer zugänglich sind. Weitere Nachteile beim Einsatz von Vinylaldehyden sind ihre Hydrolysenempfindlichkeit und ihre Instabilität. Die Vinylaldehyde sind in technischem Maßstab nur schlecht handhabbar und können in befriedigenden Ausbeuten nur aus dem teuren Vinylbutylether erhalten werden (vgl. J. Org. Chem. 36, 3390 (1971) und EP-OS-31 041).

Es wurde nun gefunden, daß man halogenierte 3,3-Dimethyl-5-hexan-2-one der Formel (I)

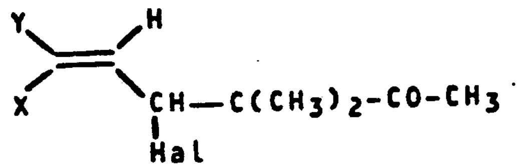

(I)

in welcher
X für Halogen steht,
Y für Halogen oder Trihalogenalkyl steht und Hal für Chlor oder Brom steht,
erhält, indem man Essigsäureester der Formel (II)

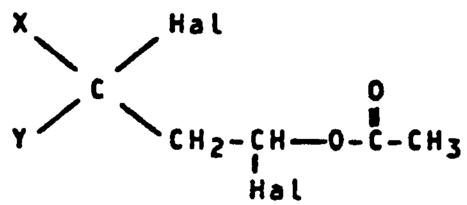

(II)

in welcher
X, Y und Hal die oben angegebenen Bedeutungen haben,
mit 2-Methylbutan-3-on-der Formel (III)

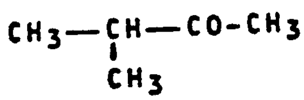

(III)

in Gegenwart wäßriger Halogenwasserstoffsäuren und gegebenenfalls in Gegenwart von zusätzlichen inerten Verdünnungsmitteln umsetzt.

Überraschenderweise lassen sich die Verbindungen der allgemeinen Formel (I) in glatten Reaktionen auf einfache weise herstellen, wobei hervorragende Ausbeuten erhalten werden. Da die einzelnen Zwischenprodukte, stabil sind und vor allem im Falle ihrer Isolierung über längere Zeit bevorratet werden können, erlaubt das erfindungsgemäße Verfahren darüberhinaus eine außerordentliche Flexibilität in der Produktion, so daß bei rasch einsetzendem Bedarf der Endprodukte eine bedarfsgerechte Fertigung möglich ist, was insbesondere durch die klimatisch bedingten starken saisonalen Schwankungen auf dem Pflanzenschutzgebiet von sehr großer Bedeutung sein kann.

Überraschenderweise läßt sich, die Essigesterverbindung der Formel (II) in höherer Ausbeute mit 2-Methylbutan-3-on der Formel III umsetzen als entsprechende Vinylaldehyde. Bei der erfindungsgemäßen Umsetzung wird die Aldehydstufe nicht durchlaufen.

Verwendet man beispielsweise Essigsäure-1,3,3,3-tetrachlorpropylester und 2-Methylbutan-3-on als Ausgangsstoffe, so kann der Reaktionsablauf durch das folgende Formelschema wiedergegeben werden:

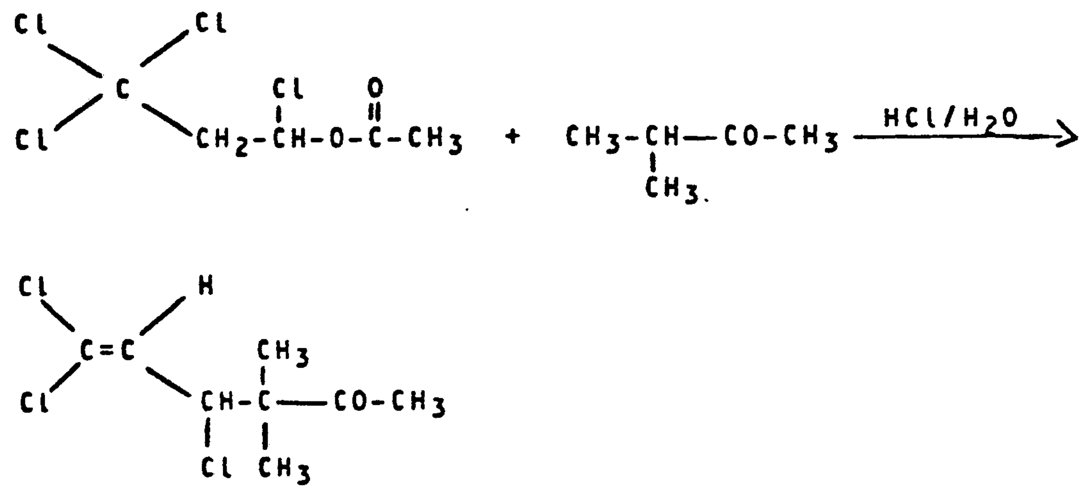


Die beim erfindungsgemäßen Verfahren zu verwendenden Essigsäureester sind durch die Formel (II) allgemein definiert. In dieser Formel steht vorzugsweise

X für Fluor, Chlor oder Brom,

Y für Fluor, Chlor, Brom oder Halogen-$C_1C_4$-Alkyl und

Hal für Chlor oder Brom.

Besonders bevorzugt sind die Verbindungen der Formel (II) in welcher

X für Fluor, Chlor oder Brom steht,

Y für Fluor, Chlor, Brom oder Trifluormethyl steht und

Hal für Chlor oder Brom steht.

Die Verbindungen der Formel (II) sind bekannt und/oder können nach bekannten Methoden hergestellt werden, indem man z. B. die Verbindungen der Formel (IV)

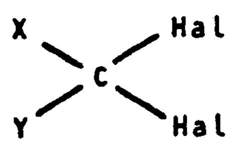

(IV)

in welcher

X, Y und Hal die oben angegebenen Bedeutungen haben,

mit Vinylacetat der Formel (V),

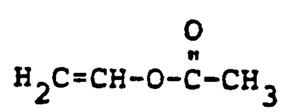

(V)

in Gegenwart von Katalysatoren, die für die Additionen solcher Verbindungen an Olefine üblicherweise verwendet werden, wie z. B. Peroxide oder andere Radikalbildner wie z. B. Azo-bis-isobutyronitril, oder Kupfer-(I)-chlorid oder Redox-Katalysatoren, in Gegenwart von inerten Verdünnungsmitteln, wie z. B., Acetonitril, bei Temperaturen zwischen 40°C und 130°C umsetzt (vgl. J. Chem. Soc. 1963, 1887 und Herstellungsbeispiel).

Als Beispiele für die Verbindungen der Formel (II) seien beispielsweise genannt:

Essigsäure-1,3,3,3-tetrachlor-, -1-brom-3,3,3-trichlor-, -1,3,3,3-tetrabrom- und -1,3-dibrom-3,3-difluor-propylester sowie Essigsäure-1,3,3-trichlor-4,4,4-trifluor-butylester.

Die Verbindungen der Formel (III), (IV) und (V) sind bekannte Verbindungen der organischen Chemie

Es kann mit oder ohne zusätzliche Verdünnungsmittel gearbeitet, werden. Als Verdünnungsmittel kommen alle gegen Chlor- oder Bromwasserstoffsäure inerten Verdünnungsmittel in Frage, wie beispielsweise Kohlenwasserstoffe, wie Cyclohexan, Petrolether, Benzol, Toluol oder Chlorkohlenwasserstoffe, wie Methylenchlorid, Chloroform, Tetrachlorkohlenstoff oder Chlorbenzol; Ether, wie Diethylether, Diisopropylether, Tetrahydrofuran, Dioxan oder 1,2-Dimethoxyethan und Säuren, wie Essigsäure.

Bei Verwendung von mit Wasser praktisch nicht mischbaren Lösungsmitteln werden vorzugsweise Phasentransfer-Katalysatoren aus der Reihe der Tetraalkyl- oder Trialkylaralkylammoniumsalze, wie z. B. Tetrabutylammoniumbromid oder Triethylbenzylammoniumchlorid eingesetzt.

Vorzugsweise wird ohne zusätzliche Verdünnungsmittel gearbeitet.

Das erfindungsgemäße Verfahren wird in Gegenwart überschüssiger Chlor- oder Bromwasserstoffsäure

durchgeführt.

Die Reaktionstemperaturen können in einem größeren Bereich variiert werden, jedoch gelingt die Reaktion überraschenderweise bereits unter äußerst schonenden Bedingungen. Im allgemeinen arbeitet man zwischen 0°C und 100°C, vorzugsweise jedoch zwischen 30°C und 80°C.

Bei der Durchführung des erfindungsgemäßen Verfahrens werden die Ausgangsstoffe (II) und (III) gewöhnlich in äquimolaren Mengen eingesetzt. Ein Überschuß an 2-Methylbutan-3-on der Formel (III) ist jedoch auch möglich. Im allgemeinen wird, bei der Durchführung des erfindungsgemäßen Verfahrens vorzugsweise die Halogenwasserstoffsäure zu einer Mischung aus (II) und (III) gegeben. Grundsätzlich kann jedoch auch umgekehrt verfahren werden. Die Isolierung der erfindungsgemäßen Verbindungen der Formel (I) geschieht nach üblichen Methoden durch Extraktion und Destillation.

Die Verbindungen der Formel (I) sind wichtige Zwischenprodukte zur Herstellung von 3-Vinyl-2,2-dimethyl-cyclopropancarbonsäuren bzw. ihren Estern, welche Verbindungen mit hoher insektizider Potenz sind (vgl. z. B. DE-OS-2 706 184 und 2 738 150).

Die Verbindungen der Formel (I) können z. B. wie folgt zu 3-Vinyl-2,2-dimethyl-cyclopropancarbonsäuren bzw. ihren Estern umgesetzt werden:

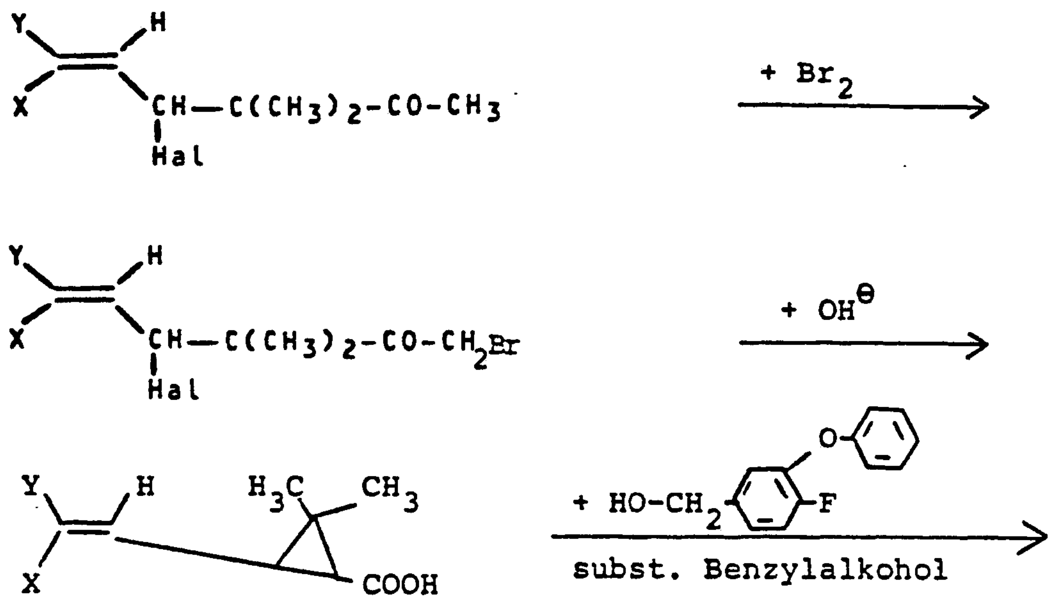


Cyclopropancarbonsäure

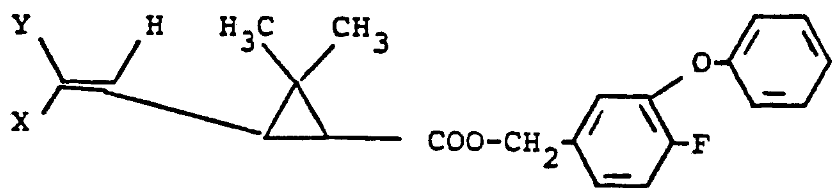

Pyrethroid-Wirkstoff

**Herstellungsbeispiele**

**Beispiel 1**

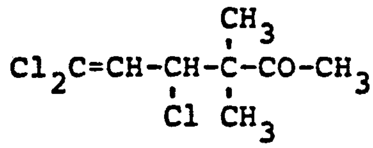

Eine Mischung aus 24 g (0,1 Mol) Essigsäure-1,3,3,3-tetrachlorpropylester und 9,5 g (0,11 Mol) Methylisopropylketon wird unter Rühren auf 55°C erhitzt und 100 ml konzentrierter Salzsäure zugetropft. Man rührt 12 Stunden bei 55°C, kühlt ab und verdünnt mit Wasser. Die Reaktionslösung wird dreimal mit Methylenchlorid extrahiert, die organische Phase mit Wasser gewaschen, getrocknet und eingeengt. Durch Destillation im Hochvakuum kann das Keton gereinigt werden.

Man erhält 21,1 g (91,9 % der Theorie) 4,6,6-Trichlor-3,3-dimethyl-5-hexen-2-on vom Siedepunkt Kp: 85°C/0,1 mbar als gelbes Öl.

**Beispiel 2**

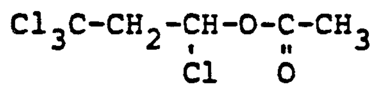

740 g Tetrachlorkohlenstoff werden unter Rühren Auf 75 bis 80°C erhitzt. Anschließend wird eine Mischung von 207 g Vinylacetat und 2 g Azo-bis-isobutyronitril in 275 g Tetrachlorkohlenstoff innerhalb von 20 Stunden zugetropft und 2 Stunden nachgerührt. Nach dem Abkühlen auf 20°C wird mit 10-%-iger wäßriger Natriumbicarbonatlösung gewaschen und über Natriumsulfat getrocknet. Tetrachlorkohlenstoff wird destillativ entfernt und der Rückstand im Hochvakuum fraktioniert.

Man erhält 160 g (24,5 % der Theorie) Essigsäure-1,3,3,3-tetrachlor-propylester vom Siedepunkt Kp: 64-65°C/2 mbar.

**Patentansprüche**

1. Verfahren zur Herstellung von halogenierten 3,3-Dimethyl-5-hexen-2-onen der Formel (I)

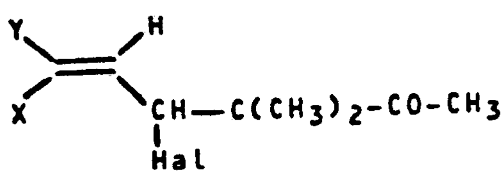

(I)

in welcher

X für Halogen steht,

Y für Halogen oder Trihalogenalkyl steht und

Hal für Chlor oder Brom steht,

dadurch gekennzeichnet, daß man Essigsäureester der Formel (II)

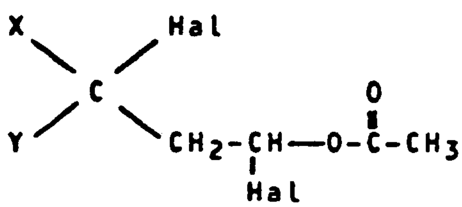

(II)

in welcher

X, Y und Hal die oben angegebenen Bedeutung haben,

mit 2-Methylbutan-3-on der Formel (III)

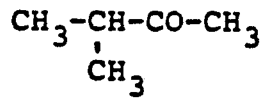

(III)

in Gegenwart wäßrigen Halogenwasserstoffsäuren und gegebenenfalls in Gegenwart von zusätzlichen inerten Verdünnungsmitteln umsetzt.

2. Verfahren gemäß Anspruch 1 zur Herstellung von Verbindungen der Formel (I), in welcher
X = Flour, Chlor oder Brom,
Y = Flour, Chlor, Brom oder Halogen-$C_1$-$C_4$-Alkyl und
Hal = Chlor oder Brom.

3. Verfahren gemäß Anspruch 1 zur Herstellung von Verbindungen der Formel (I), in welcher
X = Flour, Chlor oder Brom,
Y = Flour, Chlor, Brom oder Triflourmethyl und
Hal = Chlor oder Brom.

4. Verfahren gemäß Anspruch 1 bis 3, dadurch gekennzeichnet, daß ohne zusätzliche inerte Verdünnungsmittel gearbeitet wird.

5. Verfahren gemäß Anspruch 1 bis 4, dadurch gekennzeichnet, daß in Gegenwart überschüssiger Chlor-oder Bromwasserstoffsäuren gearbeitet wird.

6. Verfahren gemäß Anspruch 1 bis 5, dadurch gekennzeichnet, daß die Umsetzung zwischen 0°C und 100°C durchgeführt wird.

7. Verfahren gemäß Anspruch 1 bis 6, dadurch gekennzeichnet, daß die Umsetzung zwischen 30°C und 80°C durchgeführt wird.

8. Verfahren gemäß Anspruch 1 bis 7, dadurch gekennzeichnet, daß man die Ausgangsstoffe (II) und (III) in äquimolaren Mengen einsetzt.

## Claims

1. Process for the preparation of halogenated 3,3-dimethyl-5-hexen-2-ones of the formula (I)

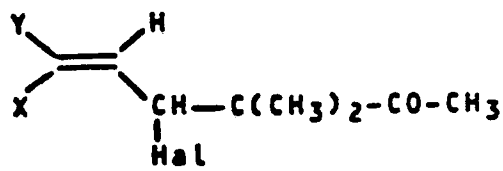

(I)

in which
X represents halogen,
Y represents halogen or trihalogenoalkyl and
Hal represents chlorine or bromine,
characterized in that acetic acid esters of the formula (II)

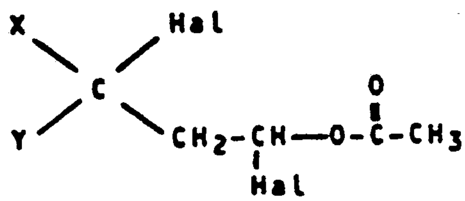

(II)

in which
X, Y and Hal have the meanings indicated above are reacted with 2-methylbutan-3-one of the formula (III)

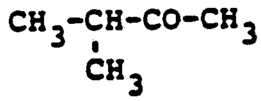
(III)

in the presence of aqueous hydrogen halide acids and, if appropriate, in the presence of additional inert diluents.

2. Process according to Claim 1 for the preparation of compounds of the formula (I)
in which
X = fluorine, chlorine or bromine,
Y = fluorine, chlorine, bromine or halogeno-$C_1$-$C_4$-alkyl and
Hal = chlorine or bromine.

3. Process according to Claim 1 for the preparation of compounds of the formula (I)
in which
X = fluorine, chlorine or bromine,
Y = fluorine, chlorine, bromine or trifluoromethyl and
Hal = chlorine or bromine.

4. Process according to Claim 1 to 3, characterized in that the reaction is carried out without additional inert diluents.

5. Process according to Claim 1 to 4, characterized in that the reaction is carried out in the presence of excess hydrochloric or hydrobromic acids.

6. Process according to Claim 1 to 5, characterized in that the reaction is carried out between 0° C and 100° C.

7. Process according to Claim 1 to 6, characterized in that the reaction is carried out between 30° C and 80° C.

8. Process according to Claim 1 to 7, characterized in that the starting materials (II) and (III) are employed in equimolar amounts.

## Revendications

1. Procédé de fabrication de 3,3-diméthyl-5-hexène-2-ones halogénées de formule (I)

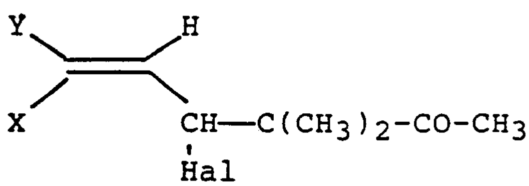
(I)

dans laquelle
X représente de l'halogène
Y de l'halogène ou un trihalogénoalcoyle et
Hal du chlore ou du brome,
caractérisé en ce qu'on fait réagir l'ester acétique de formule (II)

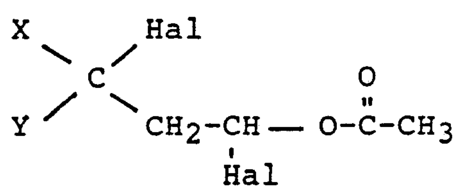
(II)

dans laquelle
X, Y et Hal ont les significations indiquées plus haut,
avec de la 2-méthylbutane-3-one de formule (III)

en présence d'hydracides halogénés aqueux et éventuellement en présence de diluants inertes additionnels.

2. Procédé selon la revendication 1 pour la fabrication de composés de formule (I) dans laquelle
X est du fluor, du chlore ou du brome,
Y du fluor, du chlore, du brome ou un halogénoalcoyle en $C_1$-$C_4$ et
Hal est du chlore ou du brome.

3. Procédé selon la revendication 1 pour la fabrication de composés de formule (I) dans laquelle
X est du fluor, du chlore ou du brome
Y du fluor, du chlore, du brome ou trifluorométhyle et
Hal du chlore ou du brome.

4. Procédé selon les revendications 1 à 3, caractérisé en ce qu'on opère sans diluants inertes additionnels.

5. Procédé selon les revendications 1 à 4, caractérisé en ce qu'on opère en présence d'acides chlorhydrique ou bromhydrique en excès.

6. Procédé selon les revendications 1 à 5, caractérisé en ce qu'on exécute la réaction entre 0° C et 100° C.

7. Procédé selon les revendications 1 à 6, caractérisé en ce qu'on exécute la réaction entre 30° C et 80° C.

8. Procédé selon les revendications 1 à 7, caractérisé en ce qu'on utilise les matières premières (II) et (III) en quantités équimolaires.